# EUROPÄISCHE PATENTSCHRIFT

(11) **EP 1 106 173 B1**
(45) Veröffentlichungstag und Bekanntmachung des Hinweises auf die Patenterteilung: **01.06.2005**
(21) Anmeldenummer: 00121183.8
(22) Anmeldetag: 29.09.2000
(51) Int. Cl.: A61K 7/50, A61K 7/48

(54) **Maiskolbenmehl als Abrasivum für Reinigungsmittel**
Corn cobs as abrasive agent in cleaning products
Epis de mais comme agent abrasif dans les produits de nettoyage

(30) Priorität: 08.12.1999 DE 19959238
(43) Veröffentlichungstag der Anmeldung: 13.06.2001
(73) Patentinhaber: Peter Greven Fett-Chemie GmbH & Co.KG, 53902 Bad Münstereifel (DE)
(72) Erfinder: Stolz, Hermann-Josef, Dr., 53902 Bad Münstereifel (DE); Börnicke, Robert, 53902 Bad Münstereifel (DE)
(74) Vertreter: von Kreisler, Alek, Dipl.-Chem.

(56) Entgegenhaltungen:
- WO-A-94/25001
- DE-A- 4 038 076
- PATENT ABSTRACTS OF JAPAN vol. 1999, no. 01, 29. Januar 1999 (1999-01-29) & JP 10 279470 A (NICHIDEN KAGAKU KK), 20. Oktober 1998 (1998-10-20)

## Beschreibung

Die Erfindung betrifft ein neues Abrasivum für Reinigungsmittel. Ein ideales Abrasivum soll abgerundete Körner haben, eine spezifische Dichte von weniger als 1 g/cm³ und biologisch abbaubar sein.

Die zuerst verwendeten Abrasiva waren auf Basis von Quarzsand und Bimsmehl. Danach sind Abrasiva mit Holzmehl entwickelt worden, die dann durch Kunststoffgranulate ersetzt wurden. In der letzten Zeit verwendet man zunehmend Bioreibkörper.

Quarzsand und Bimsmehl haben den Nachteil, daß die Körner hart und scharfkantig sind und eine Dichte von mehr als 1 g/cm³ aufweisen. Die Scharfkantigkeit der Körner hat zur Folge, daß Quarzsand und Bimsmehl eine relativ schlechte Hautverträglichkeit haben. Die relativ hohe Dichte hat den Nachteil, daß die Quarzsand- und Bimsmehle zur Verstopfung von Abflüssen durch Sedimentation führen können.

Deshalb verwendete man zunehmend Holzmehle. Die Holzmehle sind bezüglich der Hautverträglichkeit wesentlich besser als Quarzsand und Bimsmehl, und da sie eine spezifische Dichte von weniger als 1 g/cm³ haben, ist eine Verstopfung von Abflüssen durch Sedimentation nicht mehr zu befürchten. Nachteilig bei der Verwendung von Holzmehlen ist der mögliche Gehalt an Kolophonium und bestimmten Terpenen, die zu Allergien führen können. Ein weiterer Nachteil ist, daß Holzstaub im Verdacht steht, krebserregend zu sein, so daß strenge Sicherheitsvorkehrungen bei der Herstellung von Holzmehlen zu beachten sind.

Heute werden die meisten Abrasiva auf der Basis von Polyurethan- oder Polyethylenmehl hergestellt. Beide Mehle sind sehr vorteilhaft, da sie eine wenig scharfkantige Struktur haben, eine Dichte, die weniger als 1 g/cm³ beträgt, eine einstellbare Kornverteilung, eine einstellbare physikalische Härte und eine konstante Produktqualität durch reproduzierbare Herstellungsprozesse aufweisen. Allerdings sind Kunststoffgranulate sehr schwer biologisch abbaubar und können nur thermisch verwertet werden. Kunststoffmehle sind daher in fast allen Belangen ideale Abrasiva, haben aber Nachteile in bezug auf Entsorgung, Umweltschutz und auch Abbaubarkeit.

Bei der Suche nach Alternativen für biologisch abbaubare Stoffe, und insbesondere Naturstoffe, werden Bioreibköper auf Basis von Naturschalenmehl verwendet. Walnußschalenmehl weist eine relativ hohe Dichte auf, die nahe bei 1 g/cm³ liegt. Eine Sedimentation in Abflüssen kann nur durch optimierte Kornverteilung vermieden werden. Wainußschalenmehl weist außerdem ein hohes Allergiepotential auf. Die Verwendung von Nußschalen- und Kernmehlen als Abrasivum für kosmetische Zwecke ist in EP 0 559 696 beschrieben.

JP-10 279 470 offenbart sterilisiertes Maispulver und seine Verwendung als Abrasivum in Kosmetischen Zusammensetzungen.

Aus dem geschilderten Stand der Technik wird deutlich, daß noch immer ein dringender Bedarf an einem Abrasivum besteht, das vollkommen abbaubar ist, ein niedriges Allergiepotential aufweist, eine niedrige Dichte besitzt und daher als Alternative zum Naturschalenmehl verwendet werden kann.

Maismehl wird auch heute schon in unbehandelter Form als Abrasivum eingesetzt, besitzt jedoch erhebliche Nachteile hinsichtlich Optik, Geruch, Hygiene und Verkeimungsgefahr. Daher war die Verwendung von Maiskolbenmehl in Hautreinigungspräparaten bisher sehr begrenzt. Der vorliegenden Erfindung liegt die Aufgabe zugrunde, einen abrasiven Stoff für Hautreinigungsmittel auf Basis von Maiskolbenmehl bereitzustellen, der die in dem Stand der Technik geschilderten Nachteile nicht aufweist und neue Möglichkeiten bei der Verwendung von Maiskolbenmehl eröffnet. Insbesondere soll der abrasive Stoff gute Eigenschaften besitzen, ohne Zusatz oder gegebenenfalls nur mit einem minimalen Zusatz von weiteren chemischen Mitteln.

Diese Aufgabe wird durch einen speziell erhaltenen und zusammengesetzten abrasiven Stoff gemäß Anspruch 1 gelöst.

Es hat sich überraschenderweise herausgestellt, daß durch das Bleichen von Maiskolbenmehl ein hochwertiger abrasiver Stoff hergestellt werden kann. Die vorliegende Erfindung betrifft somit einen abrasiven Stoff für kosmetische Präparate, die gegebenenfalls Tenside, Seifen oder andere Emulgatoren, organische Lösungsmittel oder Öle sowie gegebenenfalls Verdikkungsmittel, Gerüststoffe, Rückfettungsmittel, Parfümstoffe, Konservierungsmittel, Farbstoffe und Antioxidantien enthalten, der dadurch gekennzeichnet ist, daß er gebleichtes Maiskolbenmehl enthält.

Das Maiskolbenmehl wurde bisher üblicherweise als Füllstoff verwendet. Eine Verwendung von Maiskolbenmehl ist in OS DE 39 14 969 A1, OS DE 36 24 468 A1, DE 32 07 036 C2, DE 32 44 452 C2 beschrieben. Maiskolbenmehl besitzt zwar eine zum Teil faserige Struktur, besteht im Gegensatz zum Walnußmehl aber nur zu etwa 5 bis 10% aus Lignin und ca. 90% aus Zellulose. Die Reib- oder Reinigungswirkung von Maiskolbenmehl ist geringer als beim Walnußschalenmehl. Die Reinigungswirkung ist aber immer noch ausreichend, da die Verschmutzungsgrade in der Industrie nicht mehr so hoch sind und ein gutes Hautreinigungsmittel eher eine bessere Hautverträglichkeit aufweisen muß. Das Maiskolbenmehl ist hautverträglicher als Walnußschalenmehl. Die spezifische Dichte von Maiskolbenmehl ist geringer als die spezifische Dichte von Walnußschalenmehl. Damit weist der erfindungsgemäß vorgeschlagene Stoff große Vorteile gegenüber dem Stand der Technik auf.

Das Bleichen von Maiskolbenmehl ist in dem Stand der Technik nicht beschrieben. Es konnte daher überraschend festgestellt werden, daß durch das Bleichen von Maiskolbenmehl dessen Eigenschaften in vorteilhafter Weise drastisch verändert werden. Das hängt damit zusammen, daß das Maiskolbenmehl während des Bleichvorgangs quillt. Durch das Bleichen wird nicht nur das Aussehen verbessert, sondern auch die Struktur und die Korneigenschaften werden verändert. Des weiteren werden auch die gegebenenfalls vorhandenen Keime abgetötet. Damit ist das gebleichte Maiskolbenmehl dem ungebleichten Maiskolbenmehl in mehrfacher Hinsicht überlegen.

Eine weitere Aufgabe der Erfindung ist es, ein einfaches Verfahren zur Herstellung von Abrasiva vorzuschlagen. Diese Aufgabe wurde durch das einstufige Herstellungsverfahren gemäß Anspruch 4 gelöst.

Eine weitere Aufgabe der Erfindung ist es, den erfindungsgemäßen abrasiven Stoff in Hautreinigungsmitteln zu verwenden. Diese Aufgabe wurde gemäß Anspruch 6 gelöst.

Schließlich ist es die Aufgabe der Erfindung, kosmetische Zubereitungen, insbesondere Handwasch- und Handreinigungsmittel, bereitzustellen, die den vorgenannten abrasiven Stoff enthalten. Diese Aufgabe wurde gemäß Anspruch 8 gelöst.

Nachstehend wird die Herstellung von dem erfindungsgemäßen Abrasivum beschrieben.

Zur Herstellung des erfindungsgemäßen Abrasivums können beispielsweise folgende Bestandteile verwendet werden :

| | |
|---|---|
| Wasser | 54,82% |
| Maiskolbenmehl | 10,00% |
| Xanthan | 0,20% |
| Bentonit | 1,00% |
| Wasserstoffperoxid 35% | 1,00% |
| Natronlauge 50% | 1,20% |
| Zitronensäure | 0,30% |
| Titandioxid | 0,50% |
| PEG 7-Glycerylcocoat | 2,00% |
| Cocamidopropylbetain | 9,00% |
| Natriumlaurylethersulfat 70% | 19,60% |
| Zitronenschalenduft | 0,20% |
| Bronopol 30% | 0,18% |

Wasser, Maiskolbenmehl, Xanthan und Bentonit werden gemischt (wobei der pH-Wert der Mischung ca. 7,4 beträgt). Nach der Quellung von Maiskolbenmehl wird Wasserstoffperoxid zugegeben und untergemischt. Das Bleichen wird mit Natronlauge aktiviert. Nach der Zugabe von Natronlauge liegt der pH-Wert bei ca. 11,9. Die Mischung wird so lange gerührt, bis der pH-Wert deutlich abgefallen ist und vorzugsweise weniger als 10 ist. Erst dann wird mit Zitronensäure das restliche Wasserstoffperoxid ausgetrieben. Nach der Bleichung werden der Bleichmischung die restlichen Rohstoffe zugefügt.

Es ist selbstverständlich, daß statt Natronlauge auch andere Hydroxide verwendet werden können. Statt der Zitronensäure können auch andere Säuren verwendet werden. Auch die Hilfsstoffe können beliebig variiert werden.

Das erfindungsgemäße Verfahren weist gegenüber den herkömmlichen Bleichverfahren den Vorteil auf, daß die Herstellung von dem Abrasivum in einem Schritt erfolgt. Daher muß das gebleichte Mehl nicht abfiltriert, anschließend getrocknet und dann nachbehandelt werden, was sich positiv auf Rohstoff- und Energieeinsatz auswirkt und Ressourcen schont. Alle Schritte werden erfindungsgemäß einstufig durchgeführt. Das erfindungsgemäße Verfahren kann auch für andere Naturstoffe verwendet werden, wobei die Verwendung für Maismehl große Vorteile hat, da Maismehl im Bleichprozeß quillt. Die eventuell im Rohstoff vorhandenen Keime werden durch den Prozeß vollständig abgetötet. Das wesentliche Merkmal des Verfahrens ist, daß eine Säure der Bleichmischung erst bei einem pH-Wert von ca. 10 oder weniger zugegeben wird. Damit ist sichergestellt, daß eine vollständige Zerstörung des Peroxids am Ende des Prozesses erfolgt, was durch Titration nachgewiesen werden kann.

Durch das erfindungsgemäße Verfahren lassen sich helle, saubere und kosmetisch einwandfreie Hautreinigungsmittel erhalten. Der Zusatz von Aufhellungsmitteln ist nicht notwendig. Der Gehalt an Titandioxid ist sehr gering und daher fast zu vernachlässigen. Die gebleichten Mehle sind biologisch vollständig abbaubar und weisen ein niedriges Allergiepotential auf. Die Gefahr, daß die Verwendung von Maiskolbenmehl zur Verstopfung von Abflüssen führen kann, besteht nicht.

## Patentansprüche

1. Abrasiver Stoff für kosmetische Präparate, insbesondere Hautreinigungsmittel, die gegebenenfalls Tenside, Seifen oder andere Emulgatoren, organische Lösungsmittel oder Öle sowie gegebenenfalls Verdickungsmittel, Gerüststoffe, Rückfettungsmittel, Parfümstoffe, Konservierungsmittel, Farbstoffe und Antioxidantien enthalten, **dadurch gekennzeichnet, dass** er gebleichtes, natürliches Maiskolbenmehl enthält.

2. Abrasiver Stoff nach Anspruch 1, **dadurch gekennzeichnet, dass** er einen Gehalt an gebleichtem Maiskolbenmehl von bis zu 30 Gew.-.%, vorzugsweise von 5 - 15 Gew.-%, aufweist.

3. Reinigungsmittel enthaltend den abrasiven Stoff nach Anspruch 1 oder 2.

4. Verfahren zur Herstellung des abrasiven Stoffes nach Anspruch 1 oder 2, **dadurch gekennzeichnet, dass** Wasser, Maiskolbenmehl und gegebenenfalls andere Hilfsstoffe gemischt werden, dass Wasserstoffperoxid und Alkalihydroxid, vorzugsweise Natronlauge, der Mischung zugesetzt werden, und wenn der pH-Wert der Mischung unter 10 absinkt, eine Säure zugesetzt wird, bis Wasserstoffperoxid nicht mehr nachzuweisen ist, und dann andere Hilfsstoffe der Mischung zugesetzt werden.

5. Verfahren nach Anspruch 4, **dadurch gekennzeichnet, dass** als Säure Zitronensäure verwendet wird.

6. Verwendung des abrasiven Stoffes nach einem der Ansprüche 1 bis 2 in Hautreinigungsmitteln, die gegebenenfalls Tenside, Seifen oder andere Emulgatoren, organische Lösungsmittel oder Öle sowie gegebenenfalls Verdickungsmittel, Gerüststoffe, Rückfettungsmittel, Parfümstoffe, Konservierungsmittel, Farbstoffe und Antioxidantien enthalten.

7. Kosmetische Zubereitungen, enthaltend den abrasiven Stoff nach einem der Ansprüche 1 bis 2.

8. Kosmetische Zubereitung nach Anspruch 7, **dadurch gekennzeichnet, dass** die kosmetische Zubereitung ein Handwasch- und Handreinigungsmittel ist.

## Claims

1. An abrasive material for cosmetic preparations, especially skin-cleansing agents, which optionally contain surfactants, soaps or other emulsifiers, organic solvents or oils and optionally thickening agents, builders, refatting agents, perfumes, preservatives, colorants and antioxidants, **characterized by** containing bleached natural corn-cob meal.

2. The abrasive material according to claim 1, **characterized by** having a content of bleached corn-cob meal of up to 30% by weight, preferably from 5 to 15% by weight.

3. A cleansing agent containing the abrasive material according to claim 1 or 2.

4. A process for preparing the abrasive material according to claim 1 or 2, **characterized in that** water, corn-cob meal and optionally other auxiliary agents are mixed, then hydrogen peroxide and alkali hydroxide, preferably aqueous sodium hydroxide, are added to the mixture, and if the pH value of the mixture decreases to below 10, an acid is added until hydrogen peroxide can no longer be detected, followed by adding other auxiliary agents to the mixture.

5. The process according to claim 4, **characterized in that** citric acid is used as said acid.

6. Use of the abrasive material according to any of claims 1 to 2 in skin-cleansing agents which optionally contain surfactants, soaps or other emulsifiers, organic solvents or oils and optionally thickening agents, builders, refatting agents, perfumes, preservatives, colorants and antioxidants.

7. Cosmetic formulations, containing said abrasive material according to any of claims 1 to 2.

8. The cosmetic formulation according to claim 7, **characterized in that** said cosmetic formulation is a hand-washing and hand-cleansing agent.

## Revendications

1. Substance abrasive pour des préparations cosmétiques, notamment pour des produits nettoyants de la peau, qui contiennent éventuellement des tensioactifs, des savons ou d'autres émulsifiants, des solvants ou des huiles organiques ainsi qu'éventuellement des agents épaississants, des adjuvants de détergence, des agents surgraissants, des substances parfumantes, des agents de conservation, des colorants et des antioxydants, **caractérisée en ce qu'**elle contient de la farine d'épi de maïs naturelle, blanchie.

2. Substance abrasive selon la revendication 1, **caractérisée en ce qu'**elle a une teneur en farine d'épi de maïs blanchie allant jusqu'à 30 % en poids, de préférence de 5 à 15 % en poids.

3. Produit nettoyant contenant la substance abrasive selon la revendication 1 ou 2.

4. Procédé de préparation de la substance abrasive selon la revendication 1 ou 2, **caractérisé en ce qu'**on mélange de l'eau, de la farine d'épi de maïs et éventuellement d'autres agents auxiliaires, on ajoute au mélange du peroxyde d'hydrogène et de l'hydroxyde alcalin, de préférence de l'hydroxyde de sodium, et dès que le pH du mélange baisse en dessous de 10, on ajoute un acide jusqu'à ce qu'on ne détecte plus de peroxyde d'hydrogène et on ajoute ensuite d'autres agents auxiliaires du mélange.

5. Procédé selon la revendication 4, **caractérisé en ce qu'**en tant qu'acide, on utilise de l'acide citrique.

6. Utilisation de la substance abrasive selon une des revendications 1 à 2 dans des produits nettoyants de la peau, qui contiennent éventuellement des tensioactifs, des savons ou d'autres émulsifiants, des solvants ou des huiles organiques ainsi qu'éventuellement des agents épaississants, des adjuvants de détergence, des agents surgraissants, des substances parfumantes, des agents de conservation, des colorants et des antioxydants.

7. Préparations cosmétiques, contenant la substance abrasive selon une des revendications 1 à 2.

8. Préparation cosmétique selon la revendication 7, **caractérisée en ce qu'**elle est un produit de lavage ou de nettoyage des mains.
